Europäisches Patentamt

European Patent Office

Office européen des brevets

(19)

(11) Publication number: **0 515 727 A1**

# EUROPEAN PATENT APPLICATION

(12)

(21) Application number: **91114923.5**

(22) Date of filing: **04.09.91**

(51) Int. Cl.5: **C02F 11/06**, B01J 10/00, A62D 3/00

(30) Priority: **20.05.91 US 703075**

(43) Date of publication of application:
**02.12.92 Bulletin 92/49**

(84) Designated Contracting States:
**DE FR GB IT**

(71) Applicant: **ROCKWELL INTERNATIONAL CORPORATION**
**2230 East Imperial Highway**
**El Segundo California 90245(US)**

(72) Inventor: **Guon, Jerold**
**23824 Aetna Street**
**Woodland Hills, California 91367(US)**

(74) Representative: **Wächtershäuser, Günter, Dr.**
**Tal 29**
**W-8000 München 2(DE)**

(54) **Waste treatment system.**

(57) A method of treating infectious and biological waste comprising introducing the waste and a source of oxygen into a molten salt bath maintained at an elevated temperature to produce disposable solid and gaseous reaction products.

FIGURE 1 OF 1

EP 0 515 727 A1

# BACKGROUND OF THE INVENTION

## Field of the Invention

This invention relates to a waste control process for the treatment of infectious and biological waste. It particularly relates to a molten salt process for destruction of infectious and biological waste apart from incineration or land fill while destroying biologically-active material at a significantly lower temperature than incineration. In addition, halogen, sulfur and phosphorous compounds, and metals which are present in the many forms of infectious and biological waste containers are also safely treated by the present invention.

## Related Art

Prior control and disposal of infectious and biological waste has been accomplished by incineration or land fill. Incineration of the waste often releases noxious gasses and results in incomplete destruction of the waste material. Land fills depend upon biological processes to destroy the infectious components of the waste and is not deemed, in the long term, ecologically sound.

Clearly, there is a need for a method of treating infectious and biological waste whereby the volume of such waste is reduced, the biologically-active components destroyed, and release of noxious gasses precluded.

## Summary of the Invention

In accordance with the present invention, it has been found that when an organic waste comprising infectious and biological components is treated in a molten salt at elevated temperatures, the catalytic oxidation properties of the salt melt and relatively long residence time in the salt melt permits complete destruction of the biologically-active material at a significantly lower temperature than conventional incineration.

Broadly, the method comprises introducing an organic waste containing the infectious and biological components and a source of gaseous oxygen such as pure oxygen, air or a mixture thereof, into a molten salt melt or bath comprising at least one alkali metal carbonate. The bath is maintained at a temperature of from about. 400°C to about 1400°C and a pressure within the range of from about 0.5 to 10 atmospheres to at least partially oxidize the organic waste. Complete combustion is generally preferred. Under such conditions, the volume of the organic waste is substantially reduced, and solid and gaseous combustion products are formed. The gaseous combustion products consist essentially of carbon dioxide and water vapor, and the solid products comprise the inorganic ash constituents of waste.

The molten salt may be either a single alkali metal carbonate, or a mixture of two or more alkali metal carbonates, sulfates or chlorides, and may include at least one and up to about 100 weight percent of a alkali metal carbonate.

Where it is desired to perform the combustion of the organic waste at a relatively low temperature, a low melting binary or ternary mixture of alkali metal carbonates may be utilized. For example, the ternary alkali metal carbonate eutectic consisting of 43.5, 31.5, and 25.0 mole percent of the carbonates of lithium and potassium, and sodium sulfate, respectively, melts at about 400°C. In the same mole percentages, a sodium, potassium, and lithium chloride mixture may be utilized for the control of melting point, viscosity and reaction rates. A preferred binary mixture is the sodium carbonate - potassium carbonate eutectic which melts at about 710°C. When the principle consideration is the cost of the molten salt, which is ultimately disposed of, a particularly preferred salt comprises sodium carbonate and optionally containing from 1 to 25 weight percent sodium sulfate, which may be used at a temperature between 750°C and 1000°C.

The exact pressure and temperature utilized are not critical, provided, of course, that they are so selected as to be above the melting point of the salt and below its decomposition temperature. Generally, the temperature and pressure will be within the range of from about 700°C to 1000°C and from 0.5 to 10 atmospheres.

The organic waste and a source of oxygen are introduced into the molten salt. Generally, the source of oxygen will be air in the interest of economy. Thus, the effluent gasses will also include unreacted oxygen and nitrogen. However, when it is desired to reduce the volume of gaseous products, pure oxygen can be used. Alternately, of course, oxygen-enriched air also may be utilized.

## BRIEF DESCRIPTION OF THE DRAWING

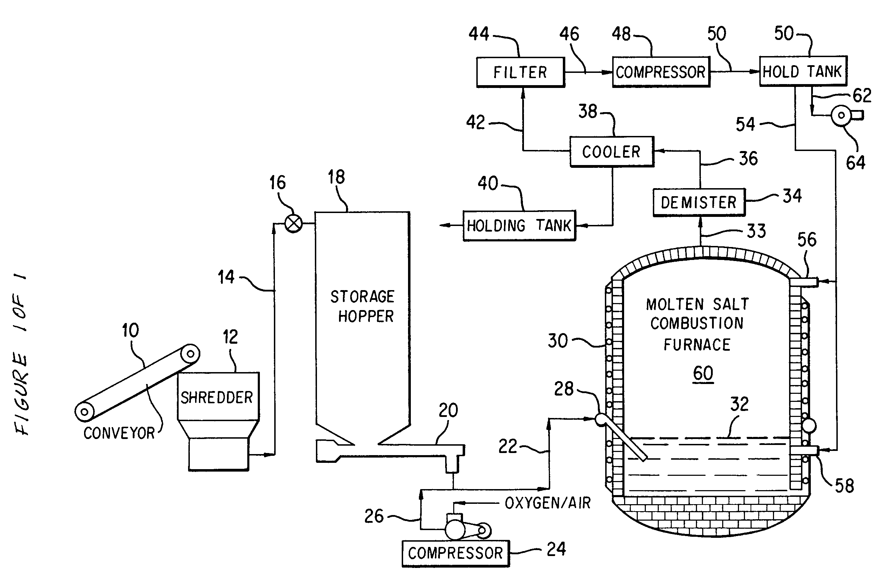

Figure 1, the sole figure of the drawing, shows a schematic illustration of a system for practicing a method of the present invention.

## Description of Preferred Embodiment

Referring to the drawing, an organic waste containing infectious and biological components is carried via a conveyor 10 to a shredder 12, which may be, for example, a hammer mill. The shredded waste then passes via a conduit 14 through a valve 16 into a storage hopper 18, provided with a screw feeder 20. From the feeder the waste passes via a conduit 22 and is mixed and conveyed with pressurized air (or oxygen) from a compressor 24 passing through a conduit 26. The mixture of air (or oxygen) and shredded waste passes through a conveyor 28 and is introduced into a molten salt combustion furnace 30, below the surface of a molten salt bath 32, which is maintained at a temperature of from about 400°C to about 1400°C, and under a pressure within the range of from about 0.5 to 10 atmospheres, to form gaseous and solid combustion products.

The gaseous combustion products consist essentially of water vapor and $CO_2$. An effluent gas, also including any unreacted oxygen from the air and the nitrogen content of the air, is withdrawn from the molten salt combustion furnace space 60 and passed through conduit 33 into demister 34 and a conduit 36 into a water cooler 38 to reduce the temperature of the gas below about 250°C. A water holding tank 40 is provided for retaining spent cooling water from water cooler 38 and water from the reaction with the waste for subsequent disposition. The cooled gasses leaving cooler 38 pass through a conduit 42 and a bag house filter 44 to remove the majority of any entrained particles. The effluent gas then passes through a conduit 46 and into a compressor 48 where the effluent gas is further treated. From the compressor, the gas is routed via conduit 50 to a hold tank 52 where it is available for recirculation into the molten salt combustion furnace by way of conduit 54. As shown in the figure, conduit 54 bifurcates into conduits 56 and 58 for introducing air back into the molten salt contained in the bottom of the furnace as well as into the gas 60 above the molten salt to enhance the combustion of the waste components.

Any excess gasses contained in the hold tank may be passed through a conduit 62, a pump 64, and to a stack (not shown) where it is vented to the atmosphere.

Advantageously, to prevent the possible escape of any airborne contaminants, conveyor 10, shredder 12, conduit 14 and hopper 18, are maintained at a subatmospheric pressure.

The following example illustrates the practice of the present invention and demonstrates its efficacy but is not intended to be construed as limiting its scope.

EXAMPLE

The following example illustrates the combustion of biologically contaminated soft paper (paper, cardboard, laboratory coats and the like), needles and syringes, bedding, small metal parts, plastic, e.g. polyvinyl chloride (PVC), glassware and similar materials. Samples of the aforementioned objects are combusted in a molten salt bed comprising 56 weight percent sodium carbonate, and 44 weight percent potassium carbonate, having a melting point of approximately 710°C. In order to prevent the formation of dioxins which would be occasioned from PVC incineration, the operating temperature is below 1200°C. During combustion, off gas is monitored continuously for carbon monoxide, carbon dioxide, hydrocarbons, oxygen, nitrous oxide, halogen, as well as dioxin. The combustion air feed rate is adjusted to about 2.5 SCSM (approximately 1.0 ft/sec superficial velocity). The feed is started and adjusted until steady state conditions are attained. The average combustion temperature is approximately 910°C. When the molten salt destruction is processed as previously disclosed, it is seen that all of the biological waste products, halogens, sulfur and phosphorous compounds, and metals are retained as stable solid disposable products for easy disposal.

**Claims**

1. A method of treating infectious and biological waste comprising:
   (a) introducing infectious and biological waste and gaseous oxygen into a molten salt bath comprising at least one alkali metal carbonate;
   b. maintaining said bath at an elevated temperature of from 400°C to 1400°C and under a pressure of from 0.5 to 10 atmospheres; and
   c. oxidizing and combusting the infectious and biological waste.

2. The method of Claim 1 wherein the molten salt bath comprises 56 weight percent sodium carbonate and 44 weight percent potassium carbonate.

3. The method of Claim 1 wherein the bath temperature is 910°C.

4. The method of Claim 1 further comprising introducing air into a combustion furnace containing the molten salt bath such that the air enters a space above the bath as well as into the bath to enhance oxidization and combustion of waste components.

FIGURE 1 OF 1

# EUROPEAN SEARCH REPORT

Application Number

EP 91 11 4923

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | FR-A-2 189 681 (ROCKWELL INTERNATIONAL CORPORATION) <br> * page 2, line 19 - page 3, line 21 * <br> * page 8, line 21 - page 9, line 30 * <br> --- | 1,3,4 | C02F11/06 <br> B01J10/00 <br> A62D3/00 |
| X | FR-A-2 350 668 (ROCKWELL INTERNATIONAL CORPORATION) <br> * page 20; claim 1 * <br> * page 1, line 20 - line 36 * <br> * page 5, line 11 - page 6, line 22; figure * | 1,3 | |
| A | --- | 2,4 | |
| X | EP-A-0 099 962 (ROCKWELL INTERNATIONAL CORPORATION) <br> * page 4, line 12 - line 31 * <br> * page 5, line 4 - line 10 * | 1,3 | |
| A | --- | 2 | |
| A | EP-A-0 070 789 (CIRTA) <br> * page 16; claims 1,3 * <br> * page 3, line 24 - line 27 * <br> ----- | 2 | TECHNICAL FIELDS SEARCHED (Int. Cl.5 ) <br><br> C02F <br> B01J <br> A62D |

The present search report has been drawn up for all claims

| Place of search | Date of completion of the search | Examiner |
|---|---|---|
| THE HAGUE | 27 AUGUST 1992 | TEPLY J. |